(19)

Europäisches
Patentamt
European
Patent Office
Office européen
des brevets

(11)  **EP 4 061 219 B1**

(12)  **EUROPEAN PATENT SPECIFICATION**

(45) Date of publication and mention
of the grant of the patent:
**28.01.2026   Bulletin 2026/05**

(21) Application number: **20889255.4**

(22) Date of filing: **18.11.2020**

(51) International Patent Classification (IPC):
*A61B 5/12* (2006.01)    *A61B 5/00* (2006.01)

(52) Cooperative Patent Classification (CPC):
**A61B 5/123**

(86) International application number:
**PCT/IB2020/000952**

(87) International publication number:
**WO 2021/099834 (27.05.2021 Gazette 2021/21)**

(54) **SCORING SPEECH AUDIOMETRY**

BEWERTUNGSSPRACHAUDIOMETRIE

NOTATION D'AUDIOMÉTRIE DE LA PAROLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **21.11.2019   US 201962938572 P**

(43) Date of publication of application:
**28.09.2022   Bulletin 2022/39**

(73) Proprietor: **Cochlear Limited
Macquarie University, New South Wales 2109
(AU)**

(72) Inventor: **VANPOUCKE, Filiep
Macquarie University, New South Wales 2109
(AU)**

(74) Representative: **Grünecker Patent- und
Rechtsanwälte
PartG mbB
Leopoldstraße 4
80802 München (DE)**

(56) References cited:
WO-A1-2019/215459    JP-A- 2014 176 582
KR-A- 20060 097 404    KR-A- 20110 090 066
US-A1- 2012 109 630    US-A1- 2015 127 347

## Description

This application is being filed on November 18, 2020, as a PCT International Patent application and claims priority to and the benefit of U.S. patent Provisional application Serial No. 62/938,572, filed November 21, 2019.

## Background

KR 2006 0097404 A discloses a mobile communication terminal capable of performing a hearing test. A voice recognition chip receives the user's voice, compares a voice-recognized word with a standard word pronunciation, and outputs a numerical value of the accuracy.
US 2015/127347 A1 discloses a method of identifying phonetically similar speech grammar terms during computer program development.
US 2012/109630 A1 discloses a text-to-speech system adapted to operate on text in a first language including sections in a second language.
WO 2019/215459 A1 discloses a computer system that analyses audio data representing a user speaking words from a body of text and identifies occasions where the user mispronounces an expected phoneme.
Audiometry relates to the measurement of one's hearing. An audiometric test can include measuring a user's reactions to sounds. For instance, a user can listen to short words in a sound booth and repeat back verbally what the user understood. In an unaided audiometric test, the user's natural hearing (e.g., without augmentation by an auditory device) is tested. In an aided audiometric test, the user's hearing as augmented by an auditory device is tested. The test can be directed to one or both of the user's ears a time. The content of the test can include monosyllabic words, disyllabic words, entire sentences, or take other forms. An audiologist can listen to and score the responses.

## Summary

A computer-readable medium according to the invention includes the features defined in claim 1.
A system according to the invention includes the features defined in claim 5 Embodiments include the features defined in the dependent claims.

## Brief Description of the Drawings

The same number represents the same element or same type of element in all drawings.

FIG. 1 illustrates an audiometric system for performing one or more audiometric tests.
FIG. 2 illustrates a method for conducting an audiometric test.
FIG. 3 illustrates an example production of a final score based on a response to a target word.
FIG. 4 illustrates another example production of a final score based on a response to a target word.
FIG. 5, which is made up of FIGS. 5A, 5B, and 5C, illustrates example audiometric test results.
FIG. 5A illustrates example audiometric test results.
FIG. 5B illustrates an example implementation of a results table.
FIG. 5C illustrates an example implementation of phoneme analysis data.
FIG. 6 illustrates an example cochlear implant system that can benefit from use of the technologies disclosed herein.
FIG. 7 is a view of an example percutaneous bone conduction device that can benefit from use of the technologies disclosed herein.
FIG. 8 illustrates an example of a computing system with which one or more of the disclosed examples can be implemented.

## Detailed Description

This application relates to audiometric testing techniques. For each target word of an audiometric test of one or more target words, a test system can audibly provide a target word to a user and receive a response of what the user audibly perceived. The response includes graphemes (e.g., the orthographic spelling of what the user perceived). The graphemes are then converted into phonemes using, for example, a dictionary or pronunciation rules of a language to produce one or more phoneme representations of the response. Then a comparison is made between the phonetic representations of the target word and the user response (e.g., using the Levinshtein algorithm to determine a distance therebetween). Where multiple potential phonetic representations exist, multiple comparisons can be made (e.g., each user response representation is compared with each target word representation) and the best matching pair is used.
The results of one or more of the comparisons can be used to determine a phoneme score. Other data in addition to or instead of phoneme scores can be used. Error patterns can be discovered across multiple comparisons of the audiometric test. For example, a person may tend to make particular kinds of errors, such as having trouble hearing vowels, fricatives, or plosives. The resulting data phoneme scores can reflect the user's ability to hear and can be the basis for one or more treatment actions if the results reveal the user may suffer from hearing loss. For instance, the treatment actions can include providing the user with a hearing device or modifying an existing hearing device of the user (e.g., adjust low frequency gains of the device responsive to vowel errors or adjusting the high frequency gains in response to errors with fricatives). Other treatment actions can include the use of rehabilitation exercises, such as playlists of words having sounds that the user had trouble hearing.

As a specific example, the target word can be "dog" and the user's response can be "do". The target word is then converted into three different phonetic pronunciations: /dɒg/, /dɔg/, /dɑg/, each relating to a different regional pronunciation of the word. The user's response ("do") is then transcribed (e.g., using a grapheme to phoneme system) into three possible phonetic forms: /dɒ/, /dɔ/ and /do:/. The resulting phonemes can be compared, with the best match being between the target word phonetic representation /dɒg/ and the user response phonetic representation /dɒ/. Comparing the two can result in a phoneme score of 67% (e.g., two of three parts match).

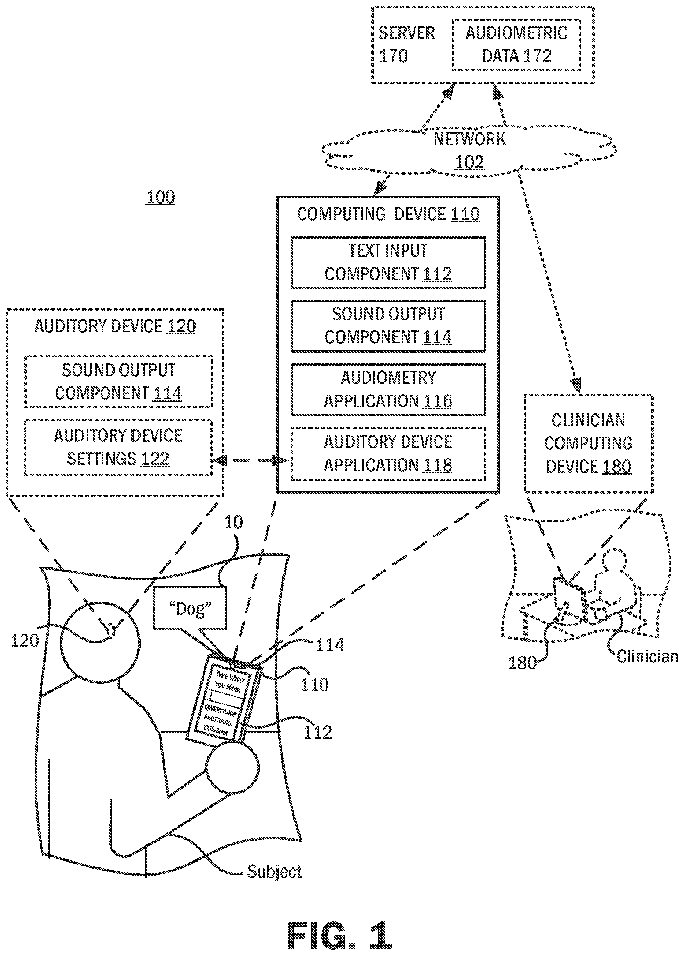

An example audiometric system for performing audiometric tests is shown in FIG. 1.

## Audiometric System

FIG. 1 illustrates an audiometric system 100 for performing one or more audiometric tests for a user. The audiometric system 100 includes a computing device 110 configured to perform the audiometry. The audiometric system 100 can further include a server 170 connected to the computing device 110 via a network 102. The audiometric system 100 can further include a clinician computing device 180. In some examples, the user of the audiometric system 100 can be the recipient of an auditory device 120.

The network 102 is a computer network, such as the Internet, that facilitates the electronic communication of data among computing devices connected to the network 102.

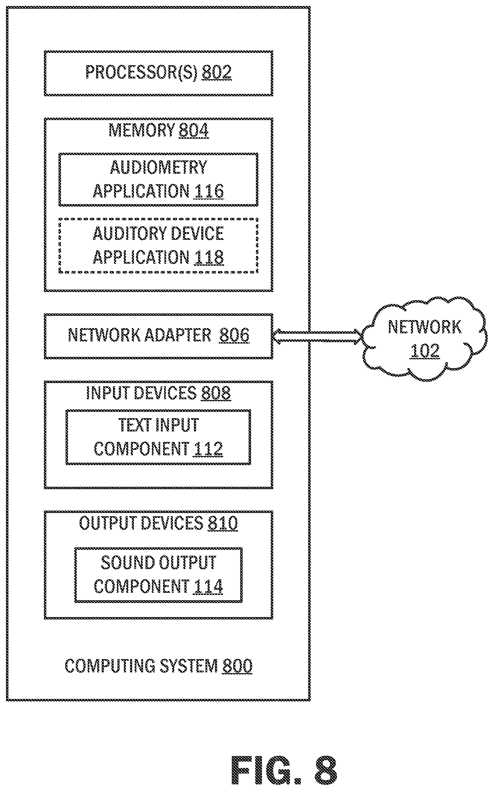

The computing device 110 can be a device having a computer functionality. The computing device 110 can be a consumer computing device owned or primarily used by the user or a parent or caregiver of the user, such as, such as a phone, tablet, laptop computer, desktop computer, consumer augmented reality device, consumer virtual reality device, smart watch, or consumer heart rate monitor, among other devices. In other examples, the computing device 110 can be a device owned or used by an organization, such as a school, clinic, or audiometry service. As illustrated, the computing device 110 can include a text input component 112, a sound output component 114, and an audiometry application 116. Where the user has an auditory device 120, the computing device 110 can include an auditory device application 118. The computing device 110 can include or more components or functionality described in relation to the computing system 800 of FIG. 8.

The text input component 112 can be a component over which the computing device 110 can receive text data from the user, such as a touchscreen configured to receive touch input (e.g., the computing device 110 can convert the touch input into text data). Where the text input component 112 includes a touchscreen, the text input component 112 can display a virtual keyboard and convert tap or swipe touch input at the keys of the virtual keyboard into text data. In another example, the text input component 112 is one or more buttons (e.g., keys of a keyboard) configured to receive button input and convert the button input into text data. In another example, the text input component 112 is a component (e.g., a microphone) of a speech-to-text system provided by the computing device 110. The computing device 110 can be configured such that applications running on the computing device 110 can receive text input via the text input component 112.

The sound output component 114 can be a component over which the computing device 110 can provide sound output, such as one or more speakers. The sound output component 114 can be a component that cooperates with another device to provide sound output. For instance, the sound output component 114 can be an audio-out port of the computing device 110 or a wireless transmitter (e.g., a BLUETOOTH component) configured to connect to another device that provides audio output (e.g., a speaker, headphones, or a component of the auditory device 120). The computing device 110 can be configured such that applications running on the computing device 110 can provide audio output via the sound output component 114.

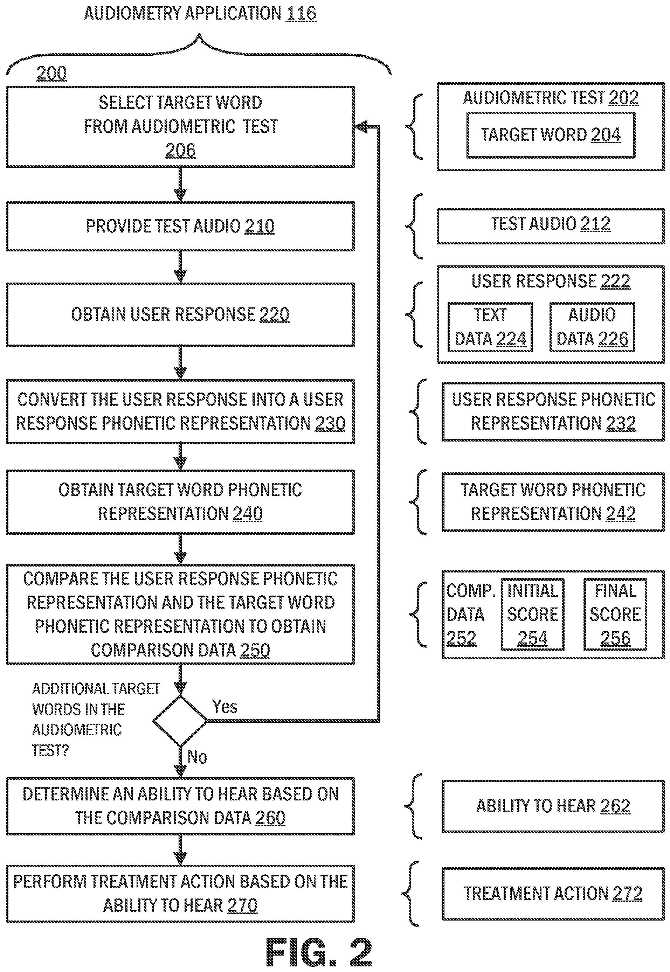

The audiometry application 116 can be software that operates on the computing device 110 and causes performance of audiometry operations described herein. In many examples, the audiometry application 116 provides audio output, receives user responses, analyzes the responses, and provides an indication of the user's ability to hear. Example operations performed by the audiometry application are described in FIG. 2. In some examples, the instructions can be obtained as part of a downloadable package, such as may be downloaded from a software distribution platform. In some examples, the audiometry application 116 is a browser via which the server 170 is accessed to provide audiometry functionality (e.g., an audiometric test web app can be provided by the server 170 and accessed by a browser of the computing device 110).

The auditory device application 118 can be software that operates on the computing device 110 and cooperates with the auditory device 120. The auditory device application 118 can be stored as computer-executable instructions in memory of the computing device 110 that, when executed, performs one or more tasks relating to the auditory device application 118. For instance, the auditory device application 118 can control the auditory device 120 (e.g., by modifying the auditory device settings 122 automatically or based on input received at the computing device 110 from the recipient), monitor usage of the auditory device 120, and obtain data from the auditory device 120. The computing device 110 can connect to the auditory device 120 via, for example, a wireless radiofrequency communication protocol (e.g., BLUETOOTH or WI-FI). The auditory device application 118 can transmit or receive data from the auditory device 120 over such a connection. The auditory device application 118 can be used to stream audio to the auditory device 120, such as from a microphone of the computing device 110 or an application running on the computing device 110 (e.g., the audiometry application 116).

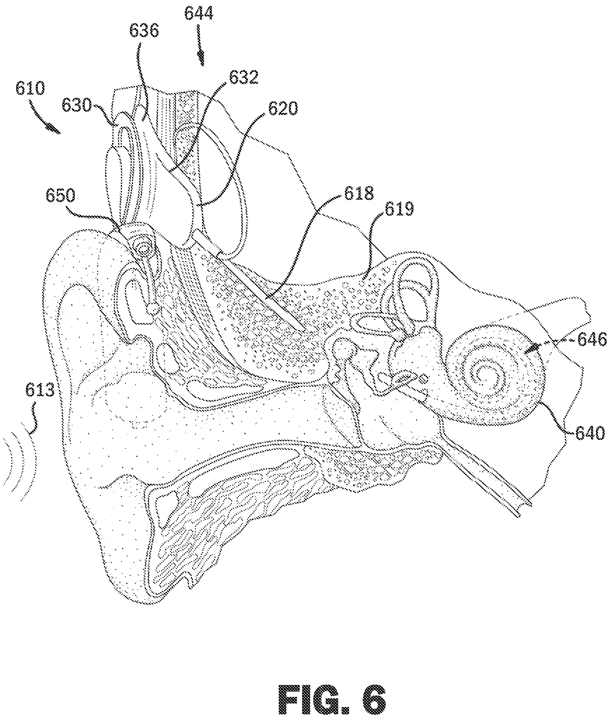

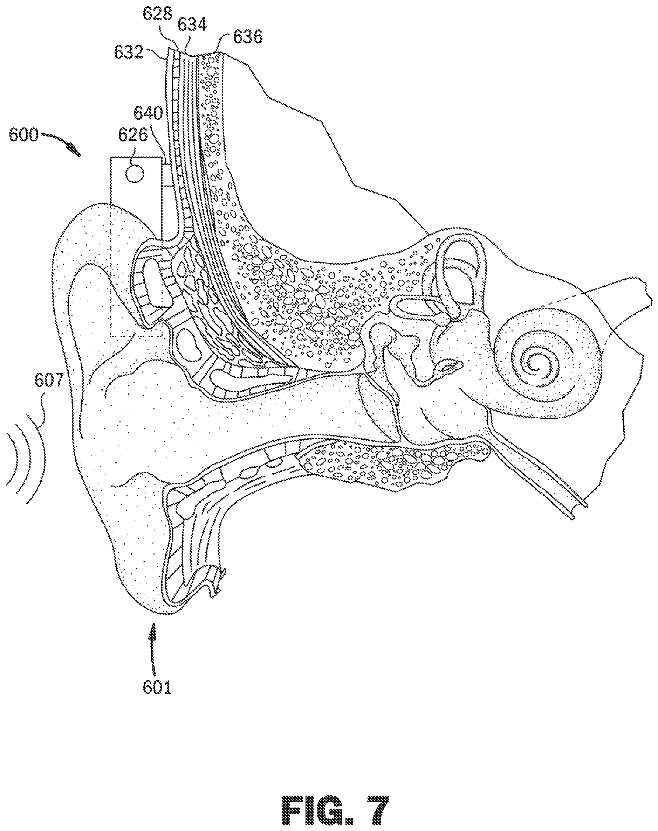

The auditory device 120 can be an apparatus relating to the user's sense of hearing. The auditory device 120 can take a variety of forms including a cochlear implant, an electroacoustic device, a percutaneous bone conduction device, a passive transcutaneous bone conduction device, an active transcutaneous bone conduction device, a middle ear device, a totally-implantable auditory device, a tinnitus management device, a mostly-implantable auditory device, an auditory brainstem implant device, a hearing aid, a tooth-anchored hearing device, a personal sound amplification product, other auditory prostheses, and combinations of the foregoing (e.g., binaural systems that include a prosthesis for a first ear of a recipient and a prosthesis of a same or different type for the second ear). Specific example implementations of the auditory device 120 are described in more detail in FIG. 6 (showing a cochlear implant) and FIG. 7 (showing a percutaneous bone conduction device). Technology disclosed herein can be used with sensory devices such as consumer auditory devices (e.g., a hearing aid or a personal sound amplification product). Further, normal hearing people or people with a hearing loss may use an auditory device 120 such as headphones or standard audiometers during a hearing test. As illustrated, the auditory device 120 can include the sound output component 114. The auditory device 120 can operate according to one or more auditory device settings 122.

The auditory device settings 122 can be one or more parameters having values that affect how the auditory device 120 operates. For example, the auditory device 120 can receive audio input from the environment (e.g., using a microphone), convert the audio input into a stimulation signal, and use the stimulation signal to produce stimulation (e.g., vibratory or electrical stimulation) to cause a hearing percept in the user. The auditory device settings 122 can include a map having minimum and maximum stimulation levels for stimulation channels. The map can then be used by the auditory device 120 to control an amount of stimulation provided. Where the auditory device 120 is a cochlear implant, the map can affect which electrodes of the cochlear implant to stimulate and in what amount based on received audio input. In some examples, the auditory device settings 122 include two or more predefined groupings of settings selectable by the recipient. The auditory device settings 122 can also include settings that modify sensory input before the sensory input is converted into a stimulation signal. Such settings can include, for example, particular audio equalizer settings can boost or cut the intensity of audio at various frequencies. In examples, auditory device settings 122 can include a minimum threshold for which received audio input causes stimulation, a maximum threshold for preventing stimulation above a level which would cause discomfort, gain parameters, intensity parameters (e.g., loudness), and compression parameters. The auditory device settings 122 can include settings that affect a dynamic range of stimulation produced by the auditory device 120. As described above, many of auditory device settings 122 affect the physical operation of the auditory device 120, such as how the auditory device 120 provides stimulation to the user in response to audio input received from the environment. Thus modifying the auditory device settings 122 can modify treatment provided by the auditory device 120. Examples of settings, settings modification, and pre-processing for auditory prostheses are described in U.S. Patent Nos. 9,473,852 and 9,338,567.

The server 170 can be a server computing device remote from the computing device 110. The server 170 can include a processing unit and memory, which are described in more detail in FIG. 8. The server 170 can further includes instructions executable to perform one or more of the operations described herein. The server 170 can be communicatively coupled to the computing device 110 via the network 102. The server 170 can be indirectly communicatively coupled to the auditory device 120 through the computing device 110 (e.g., via the auditory device application 118). In certain examples, the computing device 110 can be considered a client device of the server 170. In some examples, the functionality provided by the server 170 or the components thereof can be provided by or located on a device local to the recipient (e.g., the computing device 110 or the auditory device 120). One or both of the auditory device application 118 and the audiometry application 116 can be a client application configured to interact with the server 170. For example, as illustrated, the server 170 can include audiometric data 172, such as audiometric test data usable to provide an audiometric test. The test data can specify the kinds of tests to run, and the data used for those tests. For example, the test data can include audio files of the target words being spoken. The audio files can then be streamed to the computing device 110 or provided as downloads to the computing device 110. In some examples, the audiometric data 172 includes the results of the audiometric tests, such as for review by the clinician via the clinician computing device. In examples, audiometric test processing can be performed at the server. For instance, one or more operations described herein as being performed by the audiometry application 116 on the computing device 110 can be performed at least in part on the server 170.

The clinician computing device 180 can be a computing device used by a clinician. A clinician can be a medical professional, such as an audiologist. In an example, the clinician is a medical professional that provides care or supervision for the user. The clinician computing device 180 includes one or more software programs usable to monitor the audiometric test. For example, responsive to an audiometric test being performed for the user, the results of the test can be provided to the clinician via the clinician computing device 180 for analysis or a decision regarding how to proceed. In some examples, the clinician computing device 180 can be used by the clinician to remotely connect to the computing device 110 to administer the audiometry test.

## Method

FIG. 2 illustrates an example method 200 not part of the invention for conducting an audiometric test 202. In some examples, the method 200 can be performed as a result of one or more instructions being executed by one or more processors. For instance, the instructions can be audiometry application instructions stored on a non-transitory computer-readable medium, such as one of the computing device 110.

The audiometric test 202 can be a test of a user's hearing. The audiometric test 202 can take any of a variety of forms. The audiometric test 202 can include a list of target words 204 to be audibly provided to the user. Each target word 204 can be a particular cue to be provided to the user and to which the user provides a response (in many examples, providing an indication of what the user understood the target word to be). For instance, the target word 204 may be a consonant-vowel-consonant (CVC) word, CCVC word, CVCC word, or take another form. A CVC word need not be just a three letter word. Instead, it can be a word having a consonant sound followed by a vowel sound followed by another consonant sound. While referred to herein in the context of a "word", in certain examples, the target word 204 can be a phrase (having multiple words) or parts of a word.

The audiometric test 202 can specify the list of target words 204 in a suitable format, such as a markup language (e.g., XML). In some implementations, there need not be a predefined list of target words 204. In some examples, the content of the list of target words 204 is chosen arbitrarily. In other examples, the list or the contents of the list can be chosen for a specific reason, such as testing specific kinds of words or sounds for which the user has or is thought to have difficulty perceiving. In further examples, an audiologist selects the audiometric test 202 (e.g., remotely from the clinician computing device 180). The target word 204 can be selected from a plurality of target words of the audiometric test 202. The audiometric test 202 can be stored in a variety of locations, such as by being stored locally to the computing device 110 or at the server 170. In examples, the audiometric test 202 is downloaded to the computing device 110 from the server 170.

In examples, the audiometric test 202 can specify other data. For example, the audiometric test 202 can specify one or more complicating factors to add when providing the target words 204. For instance, an audiometric test may add particular complications to the test audio relating to particular conditions (e.g., white noise, brown noise, sounds of one or more conversations occurring, echo, reverberation, distortion, or other effects). The audiometric test 202 can further specify how the target words 204 are provided, such as at a particular volume. Further, there may be multiple different audio files containing the target word 204 (e.g., having a different accent, pronunciation, or vocal characteristics) and the audiometric test 202 can identify particular audio files to be used for the target word 204. In another example the target word 204 can be provided by a text-to-speech system and the audiometric test 202 can specify which text-to-speech system to use or parameters to be used when generating the speech (e.g., vocal characteristics).

In some examples, the method 200 can begin with operation 206.

Operation 206 includes selecting a target word 204 from an audiometric test 202. Selecting the target word 204 can include selecting a first target word 204 of the audiometric test 202 or a next target word 204 of the audiometric test 202. The selection can be in a defined order (e.g., sequentially through a list) or can be selected arbitrarily (e.g., words are selected randomly or pseudorandomly until a total number of words have been used as part of the audiometric test 202). The selecting can include selecting an identifier associated with the target word 204, an audio file associated with the target word 204, or text of the target word 204. Following operation 206, the method 200 can move to operation 210.

Operation 210 includes providing test audio 212. The test audio 212 can include the target word 204 being pronounced. Providing the test audio 212 can include causing sound to be produced based on the test audio. The test audio 212 can be provided using the sound output component 114. For instance, providing the test audio 212 can include providing the test audio 212 using a speaker, headset, or the auditory device 120 to audibly produce the test audio 212. The operation 210 can include obtaining the test audio 212, such as using an identifier of the target word 204. For instance, an identifier of the target word 204 can be used to access a file storing the test audio 212 or stream the test audio from the server 170 (e.g., from the audiometric data 172 stored thereon). In some examples, providing the test audio 212 includes generating the test audio 212. For instance, the computing device 110 can include text-to-speech functionality (e.g., using IOS's AVSpeechUtterance class or ANDROID's textToSpeech API) or a remote computing device providing text-to-speech functionality. The audiometry application 116 can provide text of the target word 204 to a text-to-speech system to cause audio output containing the target word 204 to be produced. In still other examples, a human can provide the test audio 212, such as by being prompted with the target word 204. In some examples, the test audio 212 can be processed (e.g., to have noise or distortion added). Following operation 210, the flow of the method 200 can move to operation 220.

Operation 220 includes obtaining a user response 222 from the user. The user response 222 can be obtained over a user interface. The user response 222 can include text data 224 or audio data 226. The user response 222 can be a response from the user to the test audio 212. In some examples, before, during, or after the providing of the test audio 212, the audiometry application 116 can prompt the user to provide a user response 222 describing what the user perceived or understood from the test audio 212. Obtaining the user response 222 can include providing a user interface over which the user response 222 can be received. For instance, the user interface can include a text input component 112, such as is described in FIG. 1. The operation 220 can include making the text input component 112 available for receiving text data 224 of the user response 222. The operation 220 can include making an audio input component (e.g., a microphone) available for receiving audio data 226 of the user response 222. In some examples, obtaining the user response 222 can include checking the user response 222. The checking can include, for example, determining whether the user response 222 includes a valid word. If the user response 222 includes an invalid word (e.g., a word not contained in a dictionary of the user's language), then the computing device 110 may inform the user that the word is invalid (e.g., indicate that the word is potentially misspelled). In other examples, invalid words may be allowed to be provided as input. Following operation 220, the flow of the method 200 can move to operation 230.

Operation 230 includes converting the user response 222 into a user response phonetic representation 232. As, according to the invention, the user response 222 includes text data 224, this operation 230 includes performing a grapheme to phoneme conversion on the text data 224 to generate the user response phonetic representation 232. The user response phonetic representation 232 is a representation of the user response 222 in a phonetic form. For instance, the user response phonetic representation 232 can represent the user response 222 using the ARPABET or IPA phonetic systems. ARPABET includes phonetic transcription codes that represents phonemes and allophones of general American English with distinct sequences of ASCII characters. The IPA is an alphabetic system of phonetic notation based primarily on the Latin alphabet. Other representations or combinations of representations can be used.

In an example, the conversion can be produced using one or more dictionaries. For instance, one or more dictionaries can be searched based on the text data 224 to obtain one or more user response phonetic representations 232. The one or more dictionaries can include, for example, a standard language-specific corpus dictionary (e.g., the CMU phonetic dictionary, which is a U.S. English ARPABET dictionary) or an extension dictionary that can be adjusted (e.g., by an audiologist) to extend or overrule the conversation (e.g., to correct for dialect-specific pronunciations). In an example, a data structure (e.g., a hash table, database, or dictionary data structure) can allow lookup to be performed on text input to produce one or more phonetic representations as output. In addition or instead of using one or more dictionaries, the conversion can be performed using one or more rules. The rules can be symbol substitution rules to convert the text data 224 into a phonetic transcription. For instance, the rules can reflect the standard rules in a particular language for how written from is converted into a spoken form. In some examples, the conversion can first be attempted using the one or more dictionaries and then, if one or more matches are not found in the one or more dictionaries, then conversion can be attempted using the rules.

In an example not according to the invention, where the user response 222 includes audio data 226, converting the user response 222 into a user response phonetic representation 232 can include transcribing the audio data 226 into one or more phonemes to generate the user response phonetic representation 232. For instance, a speech-to-phoneme algorithm can be used. In other examples, the audio data 226 can be transcribed into text data (e.g., using a speech-to-text process) that is then converted into phonemes using the techniques described above.

Following operation 230, the flow of the method 200 can move to operation 240.

Operation 240 includes obtaining a target word phonetic representation 242 for the target word 204. The target word phonetic representation 242 can be obtained in any of a variety of ways. For instance, the target word phonetic representation 242 can be pre-generated and stored in association with the target word 204 as part of the audiometric test 202. In such examples, the target word phonetic representation 242 can be obtained by looking up a stored phonetic representation of the target word 204. In other examples, the target word 204 is generated via a similar process to obtaining the user response phonetic representation 232. For example, a text form of the target word 204 can be used to obtain the target word phonetic representation 242 using a dictionary or a rules-based approach. Following operation 240, the flow of the method 200 can move to operation 250.

Operation 250 includes comparing the user response phonetic representation 232 and the target word phonetic representation 242 to obtain comparison data 252. In some examples, the comparison data 252 includes a score, and the operation 250 can include determining the score based on a difference between the user response phonetic representation 232 and the target word phonetic representation 242. In some examples, an initial score 254 can be determined and then modified to reach a final score 256. Where there are multiple user response phonetic representations 232 and/or target word phonetic representations 242, multiple different scores can be calculated between different pairs of the representations and a highest score can be selected as the score.

In an example, the initial score 254 can be based on whether particular parts of the phonetic representations 232, 242 match. For instance, where the target word is a consonant-vowel-consonant (CVC) word, the initial score can be broken into three components having the form $[c_1, v, c_2]$, where $c_1, v, c_2 \in \{0, 1\}$ and where a value of 1 represents a match and a value of 0 represents that there was no match. Other kinds of configurations can be used and need not be limited to three part form or CVC form.

Where the target word phonetic representation 242 is [d, ɔ, g] ("dog") and the user response phonetic representation is [d, ɪ, g] ("dig"), then the initial score 254 can be [1, 0, 1], which reflects that the phonetic portions representing the consonants of the target word 204 matched and that the phonetic portions corresponding to the vowel did not match. In an example, the insertion and substitution of additional phonemes can invalidate a correct phoneme. As a specific example, the target word phonetic representation 242 is [s, æ, p] ("sap") and the user response phonetic representation 232 is [s, n, æ, p] ("snap"). Because the consonants and vowel of the target word phonetic representation 242 are in the user response phonetic representation 232, the initial score 254 may be [1, 1, 1], but because of the addition of the extra phoneme "n" to the first consonant potion, the initial score 254 may be modified to set the first consonant portion to be incorrect. This change can result in the final score 256 being [0, 1, 1].

In some examples, the comparison data 252 includes a score based on the $[c_1, v, c_2]$ representation, such as a score s calculated as: $s = c_1 + v + c_2$ or as $S = \frac{c_1 + v + c_2}{3} \times 100$. The overall score for the audiometric test 202 can be the sum, average, or another calculation based on a combination of some or all of the individual scores of the comparison data 252 associated with each of the target words 204.

In some examples, the score or other comparison data 252 can be generated based on a distance between the user response phonetic representation 232 and the target word phonetic representation 242. The difference can be determined based on any of a variety of techniques, such as a Levenshtein distance, a Hamming distance, a Damerau-Levenshtein distance, or another distance technique. For example, where Levenshtein distance is used, the score or other comparison data can be the smallest number of deletions, insertions, and substitutions needed for the two representations 232, 242 to match. In some examples, a minimal-cost calculation is added that uses the knowledge that a phoneme is either a vowel or a consonant to determine the most likely edit operation. Where there are multiple transcriptions (e.g., multiple different possible phonetic representations for the target word 204 or the user response 222), all transcriptions can be compared and the best match (e.g., highest score) can be used. In some instances, the score or comparison data 252 can track or be based on changes in the use of consonants and vowels, such as by using the following edit indicators:

| Edit Type | Abbreviation |
| --- | --- |
| Correct Consonant | Cc |
| Correct Vowel | Cv |
| Deletion | D |
| Insert Consonant | Ic |
| Insert Vowel | Iv |
| Substitution by the same phoneme type | S |
| Substitution from vowel to consonant | Svc |
| Substitution from consonant to vowel | Scv |

The correct consonant edit type can indicate a phonetic match between a consonant portion of the user response phonetic representation 232 and the target word phonetic representation 242, such as "s" and "s". The correct vowel edit type can indicate a phonetic match between a vowel portion of the user response phonetic representation 232 and the target word phonetic representation 242, such as "ɑ" and "ɑ". The deletion edit type can indicate that the user response phonetic representation 232 entirely lacks a component (e.g., a consonant or vowel) of the target word phonetic representation. For instance, where the target word 204 is "dog", a user response phonetic representation 232 of [d, ɔ] ("do") can represent a deletion of a second consonant part ("g") of the target word phonetic representation 242 of [d, ɔ, g] ("dog"). The insert consonant edit type can indicate that the user response phonetic representation 232 adds a consonant part that is not present in the target word 204. For instance, where the target word 204 is "do", a user response phonetic representation 232 of [d, ɔ, g] ("dog") represents the addition of a second consonant part ("g") compared to the target word phonetic representation 242. The insert vowel edit type can indicate that the user response phonetic representation 232 adds a vowel part that is not present in the target word 204. For instance, where the target word phonetic representation 242 is [d,α,t] ("dot") and the user response phonetic representation 232 is [d,æ,t,ə] ("data"), the addition of the "a" phoneme can be considered an insert vowel edit. The substitution by the same phoneme type edit type can indicate that the user response phonetic representation 232 swapped one phoneme for another phoneme of the same type. For instance, in the dot-data example above, the presence of "æ" instead of "α" in the user response phonetic representation 232 can represent a substitution of the same phoneme type. The substation from vowel to consonant edit type can indicate that the user response phonetic representation 232 included a consonant where the target word phonetic representation 242 included a vowel. For instance, where the target word phonetic representation 242 is [e, ɪ,t] ("ate") and the user response phonetic representation is [k, ɪ,t] ("kit"), the substitution of "k" for "e" can represent a substitution from vowel to consonant. The substation from consonant to vowel edit type can indicate that the user response phonetic representation 232 included a vowel where the target word phonetic representation 242 included a consonant. For instance, where the target word phonetic representation 242 is [k, ɪ,t] ("kit") and the user response phonetic representation is [e, ɪ,t] ("ate"), the substitution of "e" for "k" can represent a substitution from a consonant to a vowel.

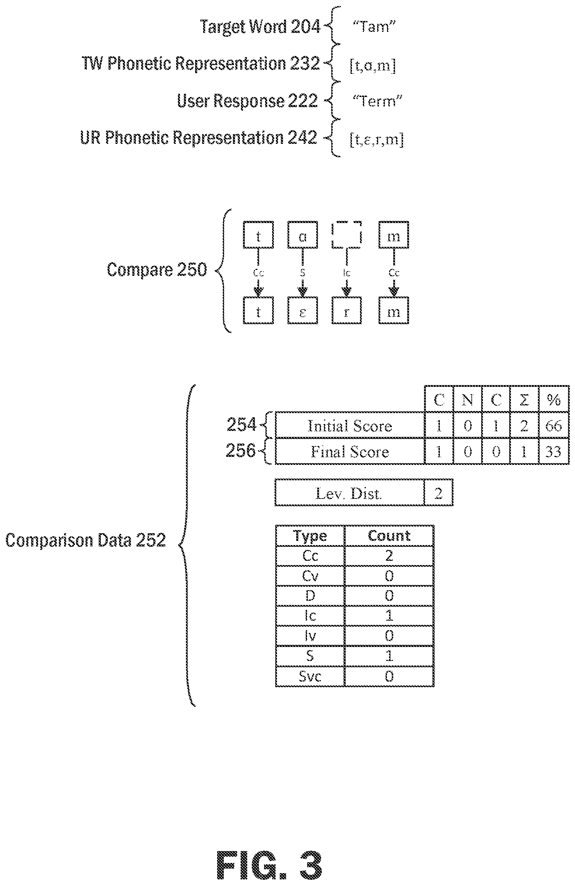

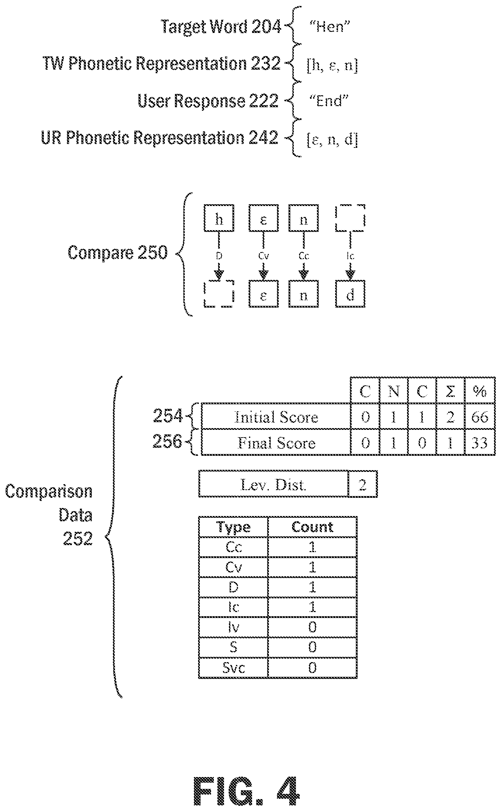

Examples of comparison data 252 resulting from the operation 250 are shown in FIGS. 3 and 4. FIG. 3 illustrates an example production of a final score 256 and other comparison data 252 based on a user response 222 to a target word 204. In this example, the target word 204 is "tam", which has a target word phonetic representation 242 of [t,α,m]. The target word 204 is provided to the user, and a user response 222 of "term" is received, which has the user response phonetic representation 232 of [t,ε,r,m]. The comparison reveals a correct consonant ("t"), a same phoneme type substitution (substituting the vowel sound "ε" for "α"), the insertion of a consonant ("r"), and a correct consonant ("m"). The comparison results in an initial score 254 of [1,0,1] because the first and last consonants match and the vowel sounds do not match. Further processing results in a final score of [1,0,0] because the last consonant was incorrect due to the insertion of "r" in addition to the correct "m". FIG. 4 illustrates another example production of a final score 256 and other comparison data 252 based on a user response 222 to a target word 204. In this example, the target word 204 is "hen" having a target word phonetic representation 242 of [h,ε,n]. The target word 204 is provided to the user, and a user response 222 of "end" is received, which has the user response phonetic representation 232 of [ε, n, d]. The comparison reveals the deletion of a consonant ("h"), a correct vowel ("ε"), a correct consonant ("n"), and the insertion of a consonant ("d"). The comparison results in an initial score 254 of [0,1,1] because the vowel and last consonant match. Further processing results in a final score 256 of [0,1,0] because the last consonant was incorrect due to the insertion of an incorrect consonant ("d") in addition to the correct consonant as part of the final consonant.

Returning to FIG. 2, as can be seen above, any of a variety of techniques can be used to compare the user response phonetic representation 232 and the target word phonetic representation 242 to generate the comparison data 252, and the comparison data 252 can include any of a variety of data. Following operation 250, the flow of the method 200 can move to operation 260. In some examples, if there are additional target words 204 in the audiometric test 202, then the flow of the method 200 can return to operation 206 for selection of a next target word 204. If there are no additional target words 204 to be provided, then the flow of the method can move to operation 260.

Operation 260 includes determining the user's ability to hear 262 based on the comparison data 252. The user's ability to hear 262 can take any of a variety of forms. In some instances, the user's ability to hear 262 can be a qualitative score, such as a sum or average of one or more scores determined in the comparison data 252. In addition or instead, the determined ability to hear 262 can include identification of particular sounds, frequencies, phonemes, syllables, parts of speech, or other aspects with which the user has difficulty hearing or understanding. In addition or instead, the ability to hear 262 can relate to an ability to hear using a particular ear or in particular conditions. For instance, an audiometric test may add particular complications to the test audio relating to particular conditions (e.g., white noise, brown noise, sounds of one or more conversations occurring, echo, reverberation, distortion, or other effects) and the ability to hear 262 can further include data regarding the conditions in which the user hears better or worse.

In some examples, the determining of the ability of the user to hear 262 is further based on the amount of time the user takes to respond to the test audio 212. For example, the method 200 can include determining an amount of time between the test audio 212 ending and beginning to receive the user response 222. The amount of time can be an indication of an amount of ease or difficulty with which the user perceived the test audio 212.

Following operation 260, the flow of the method 200 can move to operation 270.

Operation 270 includes performing a treatment action 272 based on the ability to hear 262. For instance, the treatment actions can include providing the user with a hearing device or modifying an existing hearing device of the user (e.g., adjust low frequency gains of the device responsive to vowel errors or adjusting the high frequency gains in response to errors with fricatives). In some examples, the treatment action 272 includes diagnosing the user as having a particular hearing condition.

In an example, the treatment action 272 is an action relating to the treatment of a medical condition associated with the recipient's auditory system. Various treatment actions 272 can be determined or recommended. In an example, the treatment action 272 includes reporting a performance quality of user's hearing, such as to a clinician (e.g., to help guide treatment) or caregiver (e.g., to help assure the caregiver that an auditory device of the user is functioning as intended). In an example, the treatment action 272 includes providing a metric estimating the recipient's ability to perceive particular sounds. In an example, the treatment action 272 includes recommending corrective actions.

In an example, the treatment action 272 includes recommending corrective actions (e.g., reconfiguration, reprogramming, or revising the therapy, such as by advancing to bilateral prostheses from a unilateral prosthesis). In some examples, the treatment action 272 can include recommendations or modifications for one or more auditory device settings 122 of the auditory device 120 of the user. Modifying the auditory device settings 122 can result in the modification of the ongoing treatment provided by the auditory device 120. Based on the audiometric test results 500, it can be determined that the auditory device settings 122 are sub-optimally causing auditory percepts in the recipient with respect to particular auditory input (e.g., particular phonemes or sounds) and that one or more changes to the auditory device settings 122 might be able to improve the performance of the auditory device 120. Based on the determination, information regarding the one or more changes can be provided to the recipient, a caregiver, or a clinician (e.g., by way of a report to the clinician computing device 180). In some examples, the one or more changes are automatically adopted by the auditory device 120 itself. The auditory device settings 122 are then changed, which modifies the ongoing operation of the auditory device 120. In some examples, scene-specific auditory device settings 122 are changed. For instance, the auditory device settings 122 associated with a speech mode (e.g., as determined by a scene classifier of the auditory device 120) are changed but not in other modes (e.g., music or wind modes).

The treatment action 242 can include using the ability to hear 262 or audiometric test results 500 as input to a hearing rehabilitation training program. The hearing rehabilitation program can take any of a variety of forms. For example, the hearing rehabilitation program can include the use of rehabilitation exercises, such as playlists of words having sounds that the user had trouble hearing. As a particular example, the audiometry application 116 may provide (automatically or based on clinician feedback) a user with particular exercises to perform, such as listening to a list of words starting with a particular phoneme (e.g., a particular phoneme that a hearing test indicated that the recipient had difficulty understanding).

### Example Audiometric Test Results

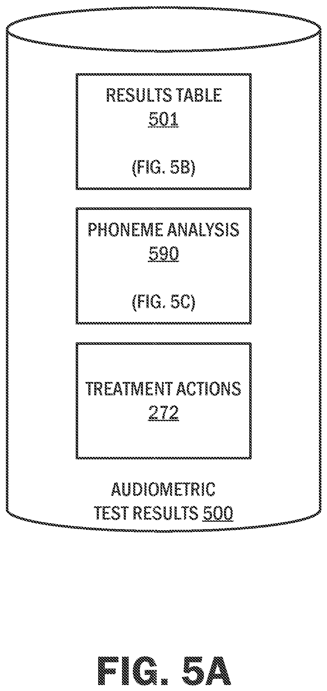

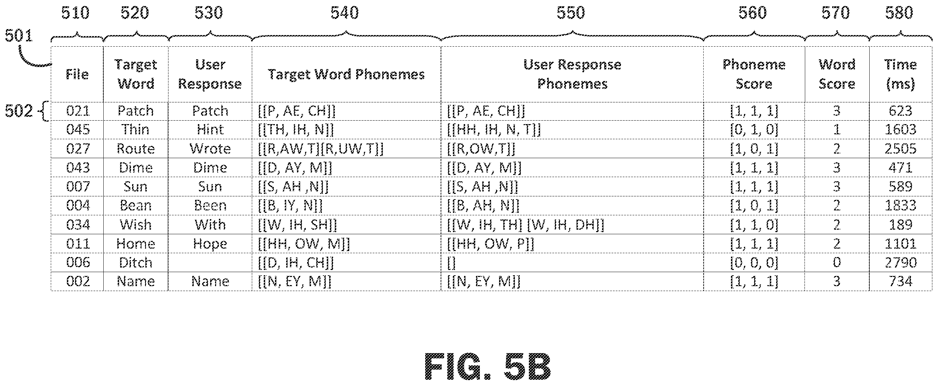

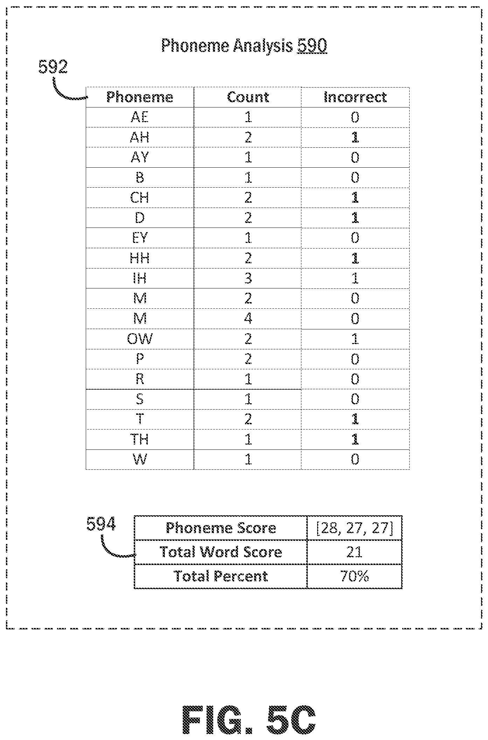

FIG. 5, which is made up of FIGS. 5A, 5B, and 5C, illustrates an example audiometric test results 500 for an audiometric test 202 (e.g., conducted using the method 200). As illustrated in FIG. 5A, the audiometric test results 500 can include a results table 501, phoneme analysis 590, and one or more treatment actions 272 that are recommended. The audiometric test results 500 can be generated, for example, as part of operations 250 and/or 260 as described above.

FIG. 5B illustrates an example implementation of the results table 501. The results table 501 can be a data table that is produced after the audiometric test 202 is provided. The results table 501 can be provided to a clinician or a program for analysis to determine an ability of the recipient to hear. The results table 501 includes ten entries 502, each corresponding to a different word-response cycle. The results table 501 further includes several fields, including a file field 510, a target word field 520, a user response field 530, a target word phoneme field 540, a user response phoneme field 550, a phoneme score field 560, a word score field 570, and a response time field 580. The file field 510 is a field describing which file (e.g., audio file) was used to produce the audio containing the target word 204. The target word field 520 describes the text of the target word 204. The user response field 530 describes the user response 222 in text form. The target word phonemes field 540 is a field that describes the target word phonetic representation 242. The user response phonemes field 550 is a field that describes the user response phonetic representation 532. As illustrated, more than one different phoneme representations can be stored in association with the phonemes fields 540, 550. The phoneme score field 560 is a field describing a phoneme score. In the illustrated example, the phoneme scores are in the three-part CVC form described above: $[c_1, v, c_2]$, where $c_1, v, c_2 \in \{0, 1\}$. Other configurations are also possible. The word score field 570 describes a word score. In the illustrated example, the word score is a count of the matches in the phoneme score field 560. The response time field 580 is a field that describes the amount of time between the target word 204 being provided and the user beginning to provide the user response. In the illustrated example, the response time field 580 describes data in the form of milliseconds.

FIG. 5C illustrates an example implementation of the phoneme analysis data 590. In the illustrated example, the phoneme analysis data 590 includes a phoneme results data 592 and score data 594.

The phoneme results data 592 can include phoneme-specific data regarding the audiometric test 202. The phoneme results data 592 can be expressed in any of a variety of ways. In the illustrated example, the phoneme results data 592 is implemented as a table showing, for each phoneme presented during the test, how correct or incorrect the user responses were with respect to a particular phoneme. Such data can be used to, for example, help identify particular sounds that the user has difficulty hearing. Certain phonemes can tend to be produced at a higher or lower frequency than others. For instance, the phoneme "IY" as pronounced in the word "bean" is relatively higher frequency than the phoneme "OW" as pronounced in the word "hope". A high incidence of incorrect answers relating to the phoneme "IY" could indicate that the user has difficulty hearing high-frequency sounds. While a high incidence of incorrect answers relating to phonemes associated with low-frequency sounds could indicate that the user has difficulty hearing low-frequency sounds.

The score data 594 can include data regarding a score of the user for the audiometric test 202. For example, as illustrated the score data 594 can include a total phoneme score in the form of a total number of correct answers for parts of CVC words in $[c_1, v, c_2]$ form. In addition or instead, the score data 594 can include a total word score (e.g., the total number of correct components) or a percent correct answers. In addition or instead, other data can be used.

## Auditory Devices

The audiometric tests described herein can be used with any of a variety of users, including users that are recipients of one or more auditory devices 120. The auditory devices 120 can include devices relating to a recipient's sense of hearing. The auditory devices 120 can take a variety of forms including a cochlear implant, an electroacoustic device, a percutaneous bone conduction device, a passive transcutaneous bone conduction device, an active transcutaneous bone conduction device, a middle ear device, a totally-implantable auditory device, a mostly-implantable auditory device, an auditory brainstem implant device, a hearing aid, a tooth-anchored hearing device, a personal sound amplification product, other auditory prostheses, and combinations of the foregoing (e.g., binaural systems that include a prosthesis for a first ear of a recipient and a prosthesis of a same or different type for the second ear).

The audiometric tests described herein can be used to determine how well the user's auditory device 120 is functioning and whether one or more changes to the auditory device settings 122 may be beneficial.

Example implementations of the auditory devices are described in more detail in FIG. 6 (showing a cochlear implant) and FIG. 7 (showing a percutaneous bone conduction device).

## Auditory Devices -Cochlear Implant System

FIG. 6 illustrates an example cochlear implant system 610 that can benefit from use of the technologies disclosed herein. The cochlear implant system 610 includes an implantable component 644 typically having an internal receiver/-transceiver unit 632, a stimulator unit 620, and an elongate lead 618. The internal receiver/transceiver unit 632 permits the cochlear implant system 610 to receive signals from and/or transmit signals to an external device 650. The external device 650 can be a button sound processor worn on the head that includes a receiver/transceiver coil 630 and sound processing components. Alternatively, the external device 650 can be just a transmitter/transceiver coil in communication with a behind-the-ear device that includes the sound processing components and microphone.

The implantable component 644 includes an internal coil 636, and preferably, a magnet (not shown) fixed relative to the internal coil 636. The magnet can be embedded in a pliable silicone or other biocompatible encapsulant, along with the internal coil 636. Signals sent generally correspond to external sound 613. The internal receiver/transceiver unit 632 and the stimulator unit 620 are hermetically sealed within a biocompatible housing, sometimes collectively referred to as a stimulator/receiver unit. Included magnets (not shown) can facilitate the operational alignment of an external coil 630 and the internal coil 636, enabling the internal coil 636 to receive power and stimulation data from the external coil 630. The external coil 630 is contained within an external portion. The elongate lead 618 has a proximal end connected to the stimulator unit 620, and a distal end 646 implanted in a cochlea 640 of the recipient. The elongate lead 618 extends from stimulator unit 620 to the cochlea 640 through a mastoid bone 619 of the recipient. The elongate lead 618 is used to provide electrical stimulation to the cochlea 640 based on the stimulation data. The stimulation data can be created based on the external sound 613 using the sound processing components and based on the auditory device settings 122.

In certain examples, the external coil 630 transmits electrical signals (e.g., power and stimulation data) to the internal coil 636 via a radio frequency (RF) link. The internal coil 636 is typically a wire antenna coil having multiple turns of electrically insulated single-strand or multi-strand platinum or gold wire. The electrical insulation of the internal coil 636 can be provided by a flexible silicone molding. Various types of energy transfer, such as infrared (IR), electromagnetic, capacitive and inductive transfer, can be used to transfer the power and/or data from external device to cochlear implant. While the above description has described internal and external coils being formed from insulated wire, in many cases, the internal and/or external coils can be implemented via electrically conductive traces.

## Auditory Devices - Percutaneous Bone Conduction Device

FIG. 7 is a view of an example of a percutaneous bone conduction device 700 that can benefit from use of the technologies disclosed herein. For example, the sensory prosthesis settings 146 of the device 700 can be customized using one or more aspects of disclosed technology. The bone conduction device 700 is positioned behind an outer ear 701 of a recipient of the device. The bone conduction device 700 includes a sound input element 726 to receive sound signals 707. The sound input element 726 can be a microphone, telecoil or similar. In the present example, the sound input element 726 may be located, for example, on or in the bone conduction device 700, or on a cable extending from the bone conduction device 700. Also, the bone conduction device 700 comprises a sound processor (not shown), a vibrating electromagnetic actuator and/or various other operational components.

More particularly, the sound input element 726 converts received sound signals into electrical signals. These electrical signals are processed by the sound processor. The sound processor generates control signals that cause the actuator to vibrate. In other words, the actuator converts the electrical signals into mechanical force to impart vibrations to a skull bone 736 of the recipient. The conversion of the electrical signals into mechanical force can be based on the sensory prosthesis settings 146, such that different sensory prosthesis settings 146 may result in different mechanical force being generated from a same sound signal 707.

The bone conduction device 700 further includes a coupling apparatus 740 to attach the bone conduction device 700 to the recipient. In the illustrated example, the coupling apparatus 740 is attached to an anchor system (not shown) implanted in the recipient. An exemplary anchor system (also referred to as a fixation system) may include a percutaneous abutment fixed to the skull bone 736. The abutment extends from the skull bone 736 through muscle 734, fat 728 and skin 732 so that the coupling apparatus 740 may be attached thereto. Such a percutaneous abutment provides an attachment location for the coupling apparatus 740 that facilitates efficient transmission of mechanical force.

## Example Computing System

FIG. 8 illustrates an example of a suitable computing system 800 with which one or more of the disclosed examples can be implemented. Computing systems, environments, or configurations that can be suitable for use with examples described herein include, but are not limited to, personal computers, server computers, hand-held devices, laptop devices, multiprocessor systems, microprocessor-based systems, programmable consumer electronics (e.g., smart phones), network PCs, minicomputers, mainframe computers, tablets, distributed computing environments that include any of the above systems or devices, and the like. The computing system 800 can be a single virtual or physical device operating in a networked environment over communication links to one or more remote devices. The remote device can be an auditory device (e.g., the auditory device 120), the computing device 110, a personal computer, a server, a router, a network personal computer, a peer device or other common network node. In examples, the computing device 110 and the server 170 includes one or more components or variations of components of the computing system 800. Further, in some examples, the auditory device 120 includes one or more components of the computing system 800.

In a basic configuration, computing system 800 includes one or more processors 802 and memory 804.

The one or more processors 802 can include one or more hardware or software processors (e.g., central processing units or microprocessors) that can obtain and execute instructions. The one or more processors 802 can communicate with and control the performance of other components of the computing system 800.

The memory 804 can include one or more software- or hardware-based computer-readable storage media operable to store information accessible by the one or more processors 802. The memory 804 can store, among other things, instructions executable by the one or more processors 802 to implement applications or cause performance of operations described herein, as well as other data. The memory 804 can be volatile memory (e.g., RAM), non-volatile memory (e.g., ROM), or combinations thereof. The memory 804 can include transitory memory or non-transitory memory. The memory 804 can also include one or more removable or nonremovable storage devices. In examples, the memory 804 can include RAM, ROM, EEPROM (Electronically-Erasable Programmable Read-Only Memory), flash memory, optical disc storage, magnetic storage, solid state storage, or any other memory media usable to store information for later access. In examples, the memory 804 encompasses a modulated data signal (e.g., a signal that has one or more of its characteristics set or changed in such a manner as to encode information in the signal), such as a carrier wave or other transport mechanism and includes any information delivery media. By way of example, and not limitation, the memory 804 can include wired media such as a wired network or direct-wired connection, and wireless media such as acoustic, RF, infrared and other wireless media or combinations thereof.

In the illustrated example, the system 800 further includes a network adapter 806, one or more input devices 808, and one or more output devices 810. The system 800 can include other components, such as a system bus, component interfaces, a graphics system, a power source (e.g., a battery), among other components.

The network adapter 806 is a component of the computing system 800 that provides network access. The network adapter 806 can provide wired or wireless network access and can support one or more of a variety of communication technologies and protocols, such as ETHERNET, cellular, BLUETOOTH, near-field communication, and RF (Radiofrequency), among others. The network adapter 806 can include one or more antennas and associated components configured for wireless communication according to one or more wireless communication technologies and protocols.

The one or more input devices 808 are devices over which the computing system 800 receives input from a user. The one or more input devices 808 can include physically-actuatable user-interface elements (e.g., buttons, switches, or dials), touch screens, keyboards, mice, pens, and voice input devices, among others input devices.

The one or more output devices 810 are devices by which the computing system 800 is able to provide output to a user. The output devices 810 can include, displays, speakers, and printers, among other output devices.

As should be appreciated, while particular uses of the technology have been illustrated and discussed above, the disclosed technology can be used with a variety of devices in accordance with many examples of the technology. The above discussion is not meant to suggest that the disclosed technology is only suitable for implementation within systems akin to that illustrated in the figures. In general, additional configurations can be used to practice the processes and systems herein and/or some aspects described can be excluded without departing from the processes and systems disclosed herein.

This disclosure described some aspects of the present technology with reference to the accompanying drawings, in which only some of the possible aspects were shown. Other aspects can, however, be embodied in many different forms and should not be construed as limited to the aspects set forth herein. Rather, these aspects were provided so that this disclosure was thorough and complete and fully conveyed the scope of the possible aspects to those skilled in the art.

As should be appreciated, the various aspects (e.g., portions, components, etc.) described with respect to the figures herein are not intended to limit the systems and processes to the particular aspects described. Accordingly, additional configurations can be used to practice the methods and systems herein and/or some aspects described can be excluded without departing from the methods and systems disclosed herein.

Similarly, where steps of a method are disclosed, those steps are described for purposes of illustrating the present methods and systems and are not intended to limit the disclosure to a particular sequence of steps. For example, the steps can be performed in differing order, two or more steps can be performed concurrently, additional steps can be performed, and disclosed steps can be excluded without departing from the present disclosure. Further, the disclosed processes can be repeated.

The scope of the invention is defined by the following claims.

**Claims**

1. A computer-readable medium storing instructions that, when executed by one or more processors of a system according to claim 5, cause the one or more processors to:

    select (206) a target word (204) from a plurality of words of an audiometric test (202);
    provide (210) test audio (212) of the target word (204) being pronounced;
    obtain (220) a user response (222) as text data (224) over a user interface;
    convert (230) the text data (224) into a user response phonetic representation (232), the conversion of the text data (224) into the user response phonetic representation (232) comprising performing a grapheme to phoneme conversion on the text data (224) to generate the user response phonetic representation (232);
    obtain (240) a target word phonetic representation (242) for the target word (204);
    compare (250) the user response phonetic representation (232) and the target word phonetic representation (242) to obtain comparison data (252); and
    determine (260) an ability of the user to hear based on the comparison data.

2. The computer-readable medium of claim 1, wherein to compare (250) the user response phonetic representation (232) and the target word phonetic representation (242) to obtain the comparison data (252) includes to:
    determine a score (254, 256) based on a difference between the user response phonetic representation (232) and the target word phonetic representation (242).

3. The computer-readable medium of claim 2, wherein to compare the user response phonetic representation (232) and the target word phonetic representation (242) to obtain the comparison data (252) includes to:
    determine the difference between the user response phonetic representation (232) and the target word phonetic representation (242) based on a Levenshtein distance between the user response phonetic representation (232) and the target word phonetic representation (242).

4. The computer-readable medium of any one of the preceding claims,
    wherein to compare (250) the user response phonetic representation (232) and the target word phonetic representation (242) includes to:

    determine an initial score (254) and modify the initial score (254) to obtain a final score (256);
    wherein the target word (204) is a consonant-vowel-consonant word;
    wherein to compare (250) the user response phonetic representation (232) and the target word phonetic representation (242) includes to
    determine a number and type of edits needed to convert the user response phonetic representation (232) to the target word phonetic representation (242) or vice versa;
    wherein to compare (250) the user response phonetic representation (232) and the target word phonetic representation (242) includes to
determine a phoneme score;
wherein the computer-readable medium is a non-transitory computer- readable medium;
wherein the computer-readable medium is a component of a phone or tablet; and/or
wherein the instructions are stored as a downloadable package.

5. A system (100) comprising:

a text input component (112);
a sound output component (114);
one or more processors (802); and
memory (804) storing instructions that, when executed by the one or more processors, cause the one or more processors to:

provide (210), via the sound output component (114), test audio (212) that includes a target word (204) being pronounced;
obtain (220) text data (224) from the text input component;
converting the text data (224) into a user response phonetic representation (232), the conversion of the text data (224) into the user response phonetic representation (232) comprising performing a grapheme to phoneme conversion on the text data (224) to generate the user response phonetic representation (232);
obtaining a target word phonetic representation (242) for the target word (204);
comparing the user response phonetic representation (232) and the target word phonetic representation (242) to obtain comparison data (252); and
determine an ability of the user to hear (252) based on the comparison data (252).

6. The system of claim 5, further comprising:
a first computing device including:

the sound output component (114);
the text input component (112);
the one or more processors (802); and
the memory (804),
wherein the memory (804) further stores instructions that, when executed, cause the one or more processors (802) to: obtain the test audio from a second computing device remote from the first computing device.

7. The system of claim 6, wherein the first computing device is a consumer computing device and wherein the second computing device is a server.

8. The system of any one of the claims 5-7,

wherein the sound output component (114) is a speaker;
wherein the sound output component (114) is wirelessly-connected to the one or more processors;
wherein the text input component (112) is a physical keyboard;
wherein the text input component (112) is a virtual keyboard; and/or
wherein the system further comprises an auditory device (120) having the sound output component (114).

**Patentansprüche**

1. Computerlesbares Medium, das Anweisungen speichert, die, wenn sie von einem oder mehreren Prozessor/en eines Systems nach Anspruch 5 ausgeführt werden, den einen oder die mehreren Prozessor/en veranlassen zum:

Auswählen (206) eines Ziel-Wortes (204) aus einer Vielzahl von Worten eines audiometrischen Tests (202);
Bereitstellen (210) von Test-Ton (212) des ausgesprochenen Ziel-Wortes (204);
Abrufen (220) einer Benutzer-Antwort (222) als Textdaten (224) über eine Benutzerschnittstelle;
Umwandeln (230) der Textdaten (224) in eine phonetische Darstellung (232) der Benutzer-Antwort, wobei die Umwandlung der Textdaten (224) in die phonetische Darstellung (232) der Benutzer-Antwort Durchführen einer Graphem-Phonem-Umwandlung an den Textdaten (224) zum Generieren der phonetischen Darstellung (232) der Benutzer-Antwort umfasst;

Erzeugen (240) einer phonetischen Darstellung (242) des Ziel-Wortes für das Ziel-Wort (204);

Vergleichen (250) der phonetischen Darstellung (232) der Benutzer-Antwort und der phonetischen Darstellung (242) des Ziel-Wortes zum Gewinnen von Vergleichsdaten (252); sowie

Bestimmen (260) einer Hörfähigkeit des Benutzers auf Basis der Vergleichsdaten.

2. Computerlesbares Medium nach Anspruch 1, wobei Vergleichen (250) der phonetischen Darstellung (232) der Benutzer-Antwort und der phonetischen Darstellung (242) des Ziel-Wortes zum Gewinnen der Vergleichsdaten (252) einschließt:

Bestimmen eines Scores (254, 256) auf Basis eines Unterschiedes zwischen der phonetischen Darstellung (232) der Benutzer-Antwort und der phonetischen Darstellung (242) des Ziel-Wortes.

3. Computerlesbares Medium nach Anspruch 2, wobei Vergleichen der phonetischen Darstellung (232) der Benutzer-Antwort und der phonetischen Darstellung (242) des Ziel-Wortes zum Gewinnen der Vergleichsdaten (252) einschließt:

Bestimmen des Unterschiedes zwischen der phonetischen Darstellung (232) der Benutzer-Antwort und der phonetischen Darstellung (242) des Ziel-Wortes auf Basis einer Levenshtein-Distanz zwischen der phonetischen Darstellung (232) der Benutzer-Antwort und der phonetischen Darstellung (242) des Ziel-Wortes.

4. Computerlesbares Medium nach einem der vorangehenden Ansprüche, wobei Vergleichen (250) der phonetischen Darstellung (232) der Benutzer-Antwort und der phonetischen Darstellung (242) des Ziel-Wortes einschließt:

Bestimmen eines Anfangs-Scores (254) und Modifizieren des Anfangs-Scores (254) zum Gewinnen eines Abschluss-Scores (256);

wobei das Ziel-Wort (204) ein Konsonant-Vokal-Konsonanten-Wort ist;

wobei Vergleichen (250) der phonetischen Darstellung (232) der Benutzer-Antwort und der phonetischen Darstellung (242) des Ziel-Wortes einschließt:

Bestimmen einer Anzahl und eines Typs von Bearbeitungsvorgängen, die erforderlich sind, um die phonetische Darstellung (232) der Benutzer-Antwort in die phonetische Darstellung (242) des Ziel-Wortes umzuwandeln oder umgekehrt;

wobei Vergleichen (250) der phonetischen Darstellung (232) der Benutzer-Antwort und der phonetischen Darstellung (242) des Ziel-Wortes einschließt:

Bestimmen eines Phonem-Scores;

wobei das computerlesbare Medium ein nichtflüchtiges computerlesbares Medium ist;

wobei das computerlesbare Medium eine Komponente eines Telefons oder Tablets ist; und/oder

wobei die Befehle als ein herunterladbares Paket gespeichert sind.

5. System (100), das umfasst:

eine Texteingabe-Komponente (112);

eine Tonausgabe-Komponente (114);

einen oder mehrere Prozessor/en (802); sowie

einen Speicher (804), der Befehle speichert, die, wenn sie von dem einen oder den mehreren Prozessor/en ausgeführt werden, den einen oder die mehreren Prozessor/en veranlassen zum:

Bereitstellen (210) von Test-Ton (212), der ein ausgesprochenes Ziel-Wort (204) einschließt, über die Tonausgabe-Komponente (114);

Abrufen (220) von Textdaten (224) von der Texteingabe-Komponente;

Umwandeln der Textdaten (224) in eine phonetische Darstellung (232) einer Benutzer-Antwort, wobei die Umwandlung der Textdaten (224) in die phonetische Darstellung (232) der Benutzer-Antwort Durchführen einer Graphem-Phonem-Umwandlung an den Textdaten (224) zum Generieren der phonetischen Darstellung (232) der Benutzer-Antwort umfasst;

Erzeugen einer phonetischen Darstellung (242) des Ziel-Wortes für das Ziel-Wort (204);

Vergleichen der phonetischen Darstellung (232) der Benutzer-Antwort und der phonetischen Darstellung (242) des Ziel-Wortes zum Gewinnen von Vergleichsdaten (252); sowie

Bestimmen einer Hörfähigkeit (252) des Benutzers auf Basis der Vergleichsdaten (252).

6.  System nach Anspruch 5, das des Weiteren umfasst:
    eine erste Computervorrichtung, die einschließt:

    die Tonausgabe-Komponente (114);
    die Texteingabe-Komponente (112);
    den einen oder die mehreren Prozessor/en (802); sowie
    den Speicher (804),
    wobei der Speicher (804) des Weiteren Befehle speichert, die, wenn sie ausgeführt werden, den einen oder die mehreren Prozessor/en (802) veranlassen, zum:
    Abrufen des Test-Tons von einer zweiten, von der ersten Computervorrichtung entfernten, Computervorrichtung.

7.  System nach Anspruch 6,
    wobei die erste Computervorrichtung eine Konsumenten-Computervorrichtung ist, und die zweite Computervorrichtung ein Server ist.

8.  System nach einem der Ansprüche 5 - 7,

    wobei die Tonausgabe-Komponente (114) ein Lautsprecher ist;
    wobei die Tonausgabe-Komponente (114) drahtlos mit dem einen oder den mehreren Prozessor/en verbunden ist;
    wobei die Texteingabe-Komponente (112) eine physische Tastatur ist;
    wobei die Texteingabe-Komponente (112) eine virtuelle Tastatur ist; und/oder
    wobei das System des Weiteren eine Hörvorrichtung (120) umfasst, die die Tonausgabe-Komponente (114) aufweist.

**Revendications**

1.  Support lisible par ordinateur stockant des instructions qui, lorsqu'elles sont exécutées par un ou plusieurs processeurs d'un système selon la revendication 5, amènent les un ou plusieurs processeurs à :

    sélectionner (206) un mot cible (204) parmi une pluralité de mots d'un test audiométrique (202) ;
    fournir (210) un audio test (212) du mot cible (204) qui est prononcé ;
    obtenir (220) une réponse utilisateur (222) sous forme de données textuelles (224) via une interface utilisateur ;
    convertir (230) les données textuelles (224) en une représentation phonétique de réponse utilisateur (232), la conversion des données textuelles (224) en la représentation phonétique de réponse utilisateur (232) comprenant la réalisation d'une conversion graphème en phonème sur les données textuelles (224) pour générer la représentation phonétique de réponse utilisateur (232) ;
    obtenir (240) une représentation phonétique de mot cible (242) pour le mot cible (204) ; comparer (250) la représentation phonétique de réponse utilisateur (232) et la représentation phonétique de mot cible (242) pour obtenir des données de comparaison (252) ; et
    déterminer (260) une capacité de l'utilisateur à entendre sur la base des données de comparaison.

2.  Support lisible par ordinateur selon la revendication 1, dans lequel la comparaison (250) de la représentation phonétique de réponse utilisateur (232) et de la représentation phonétique de mot cible (242) afin d'obtenir les données de comparaison (252) comporte :
    la détermination d'un score (254, 256) sur la base d'une différence entre la représentation phonétique de réponse utilisateur (232) et la représentation phonétique de mot cible (242).

3.  Support lisible par ordinateur selon la revendication 2, dans lequel la comparaison de la représentation phonétique de réponse utilisateur (232) et de la représentation phonétique de mot cible (242) afin d'obtenir les données de comparaison (252) comporte :
    la détermination de la différence entre la représentation phonétique de réponse utilisateur (232) et la représentation phonétique de mot cible (242) sur la base d'une distance de Levenshtein entre la représentation phonétique de réponse utilisateur (232) et la représentation phonétique de mot cible (242).

**4.** Support lisible par ordinateur selon l'une quelconque des revendications précédentes, dans lequel la comparaison (250) de la représentation phonétique de réponse utilisateur (232) et de la représentation phonétique de mot cible (242) comporte :

la détermination d'un score initial (254) et la modification du score initial (254) pour obtenir un score final (256) ;

dans lequel le mot cible (204) est un mot consonne-voyelle-consonne ;

dans lequel la comparaison (250) de la représentation phonétique de réponse utilisateur (232) et de la représentation phonétique de mot cible (242) comporte la détermination d'un nombre et d'un type de modifications nécessaires pour convertir la représentation phonétique de réponse utilisateur (232) en la représentation phonétique de mot cible (242) ou vice versa ;

dans lequel la comparaison (250) de la représentation phonétique de réponse utilisateur (232) et de la représentation phonétique de mot cible (242) comporte la détermination d'un score phonémique ;

dans lequel le support lisible par ordinateur est un support non transitoire lisible par ordinateur ;

dans lequel le support lisible par ordinateur est un composant d'un téléphone ou d'une tablette ; et/ou

dans lequel les instructions sont stockées sous forme d'un package téléchargeable.

**5.** Système (100) comprenant :

un composant de saisie de texte (112) ;

un composant de sortie sonore (114) ;

un ou plusieurs processeurs (802) ; et

une mémoire (804) stockant des instructions qui, lorsqu'elles sont exécutées par les un ou plusieurs processeurs, amènent les un ou plusieurs processeurs à :

fournir (210), via le composant de sortie sonore (114), un audio test (212) qui comporte un mot cible (204) qui est prononcé ;

obtenir (220) des données textuelles (224) à partir du composant de saisie de texte ;

convertir les données textuelles (224) en une représentation phonétique de réponse utilisateur (232), la conversion des données textuelles (224) en la représentation phonétique de réponse utilisateur (232) comprenant la réalisation d'une conversion graphème en phonème sur les données textuelles (224) pour générer la représentation phonétique de réponse utilisateur (232) ;

obtenir une représentation phonétique de mot cible (242) pour le mot cible (204) ;

comparer la représentation phonétique de réponse utilisateur (232) et la représentation phonétique de mot cible (242) pour obtenir des données de comparaison (252) ; et

déterminer une capacité de l'utilisateur à entendre (252) sur la base des données de comparaison (252).

**6.** Système selon la revendication 5, comprenant en outre :
un premier dispositif informatique comportant :

le composant de sortie sonore (114) ;

le composant de saisie de texte (112) ;

les un ou plusieurs processeurs (802) ; et

la mémoire (804),

dans lequel la mémoire (804) stocke en outre des instructions qui, lorsqu'elles sont exécutées, amènent les un ou plusieurs processeurs (802) à : obtenir l'audio test auprès d'un second dispositif informatique distant du premier dispositif informatique.

**7.** Système selon la revendication 6, dans lequel le premier dispositif informatique est un dispositif informatique grand public et dans lequel le second dispositif informatique est un serveur.

**8.** Système selon l'une quelconque des revendications 5 à 7,

dans lequel le composant de sortie sonore (114) est un haut-parleur ;

dans lequel le composant de sortie sonore (114) est connecté sans fil aux un ou plusieurs processeurs ;

dans lequel le composant de saisie de texte (112) est un clavier physique ;

dans lequel le composant de saisie de texte (112) est un clavier virtuel ; et/ou

dans lequel le système comprend en outre un dispositif acoustique (120) ayant le composant de sortie sonore

(114).

**FIG. 1**

AUDIOMETRY APPLICATION 116

200

SELECT TARGET WORD
FROM AUDIOMETRIC TEST
206

AUDIOMETRIC TEST 202

TARGET WORD 204

PROVIDE TEST AUDIO 210

TEST AUDIO 212

OBTAIN USER RESPONSE 220

USER RESPONSE 222

TEXT DATA 224    AUDIO DATA 226

CONVERT THE USER RESPONSE INTO A USER
RESPONSE PHONETIC REPRESENTATION 230

USER RESPONSE PHONETIC
REPRESENTATION 232

OBTAIN TARGET WORD PHONETIC
REPRESENTATION 240

TARGET WORD PHONETIC
REPRESENTATION 242

COMPARE THE USER RESPONSE PHONETIC
REPRESENTATION AND THE TARGET WORD
PHONETIC REPRESENTATION TO OBTAIN
COMPARISON DATA 250

COMP. DATA 252    INITIAL SCORE 254    FINAL SCORE 256

ADDITIONAL TARGET
WORDS IN THE
AUDIOMETRIC
TEST?               Yes

No

DETERMINE AN ABILITY TO HEAR BASED ON
THE COMPARISON DATA 260

ABILITY TO HEAR 262

PERFORM TREATMENT ACTION BASED ON THE
ABILITY TO HEAR 270

TREATMENT ACTION 272

FIG. 2

Target Word 204 ⟨ "Tam"

TW Phonetic Representation 232 ⟨ [t,ɑ,m]

User Response 222 ⟨ "Term"

UR Phonetic Representation 242 ⟨ [t,ɛ,r,m]

Compare 250 ⟨

| t | ɑ | [ ] | m |
|---|---|---|---|
| Cc | S | Ic | Cc |
| t | ɛ | r | m |

Comparison Data 252 ⟨

|  |  | C | N | C | Σ | % |
|---|---|---|---|---|---|---|
| 254 ⟨ | Initial Score | 1 | 0 | 1 | 2 | 66 |
| 256 ⟨ | Final Score | 1 | 0 | 0 | 1 | 33 |

| Lev. Dist. | 2 |
|---|---|

| Type | Count |
|---|---|
| Cc | 2 |
| Cv | 0 |
| D | 0 |
| Ic | 1 |
| Iv | 0 |
| S | 1 |
| Svc | 0 |

# FIG. 3

Target Word 204 { "Hen"

TW Phonetic Representation 232 { [h, ε, n]

User Response 222 { "End"

UR Phonetic Representation 242 { [ε, n, d]

Compare 250 {

| h | ε | n | [ ] |
|---|---|---|---|
| D | Cv | Cc | Ic |
| [ ] | ε | n | d |

Comparison Data 252 {

| | C | N | C | Σ | % |
|---|---|---|---|---|---|
| 254 { Initial Score | 0 | 1 | 1 | 2 | 66 |
| 256 { Final Score | 0 | 1 | 0 | 1 | 33 |

| Lev. Dist. | 2 |
|---|---|

| Type | Count |
|---|---|
| Cc | 1 |
| Cv | 1 |
| D | 1 |
| Ic | 1 |
| Iv | 0 |
| S | 0 |
| Svc | 0 |

# FIG. 4

RESULTS TABLE
501

(FIG. 5B)

PHONEME ANALYSIS
590

(FIG. 5C)

TREATMENT ACTIONS
272

AUDIOMETRIC
TEST RESULTS 500

# FIG. 5A

| File | Target Word | User Response | Target Word Phonemes | User Response Phonemes | Phoneme Score | Word Score | Time (ms) |
|---|---|---|---|---|---|---|---|
| 021 | Patch | Patch | [[P, AE, CH]] | [[P, AE, CH]] | [1, 1, 1] | 3 | 623 |
| 045 | Thin | Hint | [[TH, IH, N]] | [[HH, IH, N, T]] | [0, 1, 0] | 1 | 1603 |
| 027 | Route | Wrote | [[R,AW,T][R,UW,T]] | [[R,OW,T]] | [1, 0, 1] | 2 | 2505 |
| 043 | Dime | Dime | [[D, AY, M]] | [[D, AY, M]] | [1, 1, 1] | 3 | 471 |
| 007 | Sun | Sun | [[S, AH ,N]] | [[S, AH ,N]] | [1, 1, 1] | 3 | 589 |
| 004 | Bean | Been | [[B, IY, N]] | [[B, AH, N]] | [1, 0, 1] | 2 | 1833 |
| 034 | Wish | With | [[W, IH, SH]] | [[W, IH, TH] [W, IH, DH]] | [1, 1, 0] | 2 | 189 |
| 011 | Home | Hope | [[HH, OW, M]] | [[HH, OW, P]] | [1, 1, 1] | 2 | 1101 |
| 006 | Ditch | | [[D, IH, CH]] | [] | [0, 0, 0] | 0 | 2790 |
| 002 | Name | Name | [[N, EY, M]] | [[N, EY, M]] | [1, 1, 1] | 3 | 734 |

Column labels above table: 510, 520, 530, 540, 550, 560, 570, 580. Row bracket labels: 501, 502.

**FIG. 5B**

EP 4 061 219 B1

Phoneme Analysis 590

592

| Phoneme | Count | Incorrect |
|---------|-------|-----------|
| AE | 1 | 0 |
| AH | 2 | 1 |
| AY | 1 | 0 |
| B | 1 | 0 |
| CH | 2 | 1 |
| D | 2 | 1 |
| EY | 1 | 0 |
| HH | 2 | 1 |
| IH | 3 | 1 |
| M | 2 | 0 |
| M | 4 | 0 |
| OW | 2 | 1 |
| P | 2 | 0 |
| R | 1 | 0 |
| S | 1 | 0 |
| T | 2 | 1 |
| TH | 1 | 1 |
| W | 1 | 0 |

594

| | |
|---|---|
| Phoneme Score | [28, 27, 27] |
| Total Word Score | 21 |
| Total Percent | 70% |

# FIG. 5C

**FIG. 6**

**FIG. 7**

PROCESSOR(S) 802

MEMORY 804

AUDIOMETRY APPLICATION 116

AUDITORY DEVICE APPLICATION 118

NETWORK ADAPTER 806

NETWORK 102

INPUT DEVICES 808

TEXT INPUT COMPONENT 112

OUTPUT DEVICES 810

SOUND OUTPUT COMPONENT 114

COMPUTING SYSTEM 800

FIG. 8

**EP 4 061 219 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62938572
- KR 20060097404 A
- US 2015127347 A1
- US 2012109630 A1
- WO 2019215459 A1
- US 9473852 B
- US 9338567 B